# EUROPEAN PATENT APPLICATION

(11) **EP 4 563 081 A1**
(43) Date of publication of application: **04.06.2025**
(21) Application number: 23856183.1
(22) Date of filing: 06.06.2023
(51) Int. Cl.: A61B 5/0205, A47C 17/04

(54) **MILLIMETER WAVE DETECTION APPARATUS, INSTALLATION STRUCTURE, CONTROL SYSTEM, SEAT, AND CONTROL METHOD**

(30) Priority: 23.08.2022 CN 202211012042; 23.08.2022 CN 202222218395 U
(71) Applicant: DewertOkin Technology Group Co., Ltd., Jiaxing City, Zhejiang Province 314011 (CN)
(72) Inventor: LAI, Youcun, Jiaxing, Zhejiang 314011 (CN); LI, Long, Jiaxing, Zhejiang 314011 (CN); YAO, Jiaqian, Jiaxing, Zhejiang 314011 (CN)
(74) Representative: Grünecker Patent- und Rechtsanwälte PartG mbB
(86) International application number: PCT/CN2023/098631
(87) International publication number: WO 2024/041091

(57) **Abstract**

Provided are a millimeter wave detection apparatus, a mounting structure (3), a control system, a chair (100), and a control method. The millimeter wave detection apparatus includes a mounting portion (1) and a sensing portion (2). The mounting portion (1) includes a mounting housing (11) and a circuit board (12) disposed within the mounting housing (11). The sensing portion (2) includes a sensing housing (21) and a millimeter wave sensor (22) disposed within the sensing housing (21). The millimeter wave sensor (22) is electrically connected to the circuit board (12). The sensing housing (21) is provided with a sensing panel (23) corresponding to the millimeter wave sensor (22). The sensing housing (21) is disposed on the mounting housing (11) with an adjustable angle to adjust the orientation of the sensing panel (23).

## Description

This application claims priority to Chinese Patent Application No. 202211012042.8 and Chinese Patent Application No. 202222218395.5 filed with China National Intellectual Property Administration (CNIPA) on Aug. 23, 2022, the disclosures of which are incorporated herein by reference in their entireties.

### TECHNICAL FIELD

The present application relates to the technical field of smart furniture, for example, a millimeter wave detection apparatus, a mounting structure, a control system, a chair, and a control method.

### BACKGROUND

Millimeter wave sensors have the advantages of small volume, easy integration, and high spatial resolution. Compared with optical sensors such as cameras, infrared rays, and laser, the millimeter wave sensors have strong capabilities to penetrate fog, smoke, and dust and have strong anti-interference capabilities.

With the improvement in living standards, people have higher requirements for the smartness of furniture. The millimeter wave sensors have also begun to be applied in smart home appliances and are used to detect the heart rate, breathing, pulses, sleep state (including low, medium and deep sleep states), fatigue, and other vital information of users. In the related art, the mounting form of millimeter wave sensors used in smart furniture is single, and once the mounting is completed, the angle of the millimeter wave sensor cannot be changed, and the millimeter wave sensor has a low matching degree, even affecting the detection accuracy.

### SUMMARY

The present application provides a millimeter wave detection apparatus, a mounting structure, a control system, a chair, and a control method to improve the matching degree and detection accuracy of the millimeter wave detection apparatus.

A millimeter wave detection apparatus is provided. The millimeter wave detection apparatus includes a mounting portion and a sensing portion; the mounting portion includes a mounting housing and a circuit board disposed within the mounting housing; the sensing portion includes a sensing housing and a millimeter wave sensor disposed within the sensing housing; the millimeter wave sensor is electrically connected to the circuit board, the sensing housing is provided with a sensing panel corresponding to the millimeter wave sensor, and the sensing housing is disposed on the mounting housing with an adjustable angle to adjust an orientation of the sensing panel.

As an optional solution for the millimeter wave detection apparatus, an angle adjustment range of the sensing housing is from 0° to 90°.

As an optional solution for the millimeter wave detection apparatus, one side of the mounting housing is provided with an angle scale, and an angle pointer corresponding to the angle scale is provided on a first side of the sensing housing relative to the angle scale; a second side of the mounting housing relative to the angle scale is provided with a detachable adjustment connector, and the adjustment connector is configured to lock or unlock the sensing housing and the mounting housing.

As an optional solution for the millimeter wave detection apparatus, the millimeter wave detection apparatus further includes a wire electrically connected to the circuit board, an exterior of the wire is securely provided with a connection structure, the connection structure is rotatably connected to the mounting housing; and a wire fastener is disposed within the mounting housing and configured to prevent the wire from falling off.

As an optional solution for the millimeter wave detection apparatus, the millimeter wave detection apparatus further includes multiple function extension sensors, and the multiple function extension sensors are mounted within at least one of the sensing housing or the mounting housing.

As an optional solution for the millimeter wave detection apparatus, the multiple function extension sensors include at least one of the following: a first sensor installed within the mounting housing, where the mounting housing is provided with a first through hole corresponding to the first sensor; or a second sensor installed within the sensing housing, where the sensing housing is provided with a second through hole corresponding to the second sensor.

As an optional solution for the millimeter wave detection apparatus, the mounting housing is provided with a quick-mounting structure, and the quick-mounting structure is configured to secure the millimeter wave detection apparatus.

A mounting structure is provided for securing the millimeter wave detection apparatus of any one of the preceding solutions. The mounting structure includes an adapter plate and a mounting plate, the mounting plate is securely disposed at an end of the adapter plate in the width direction, and the mounting housing is detachably disposed on the mounting plate; or the mounting structure includes an adapter plate, and the mounting housing is detachably disposed on the adapter plate.

As an optional solution for the mounting structure, the mounting structure is provided with a quick-mounting member, where the quick-mounting member is securely connected to the mounting structure and configured to mate with a quick-mounting structure on the mounting housing to secure the millimeter wave detection apparatus to the mounting structure.

A smart furniture control system is provided. The smart furniture control system includes a control hub, a drive assembly, and the millimeter wave detection apparatus of any one of the preceding solutions, the millimeter wave detection apparatus is configured to transmit a sensing radio wave to a body part of a user and receive a human body reflection radio wave obtained by reflecting the sensing radio wave by the body part of the user; process the reflection radio wave to acquire vital information of the user; determine and output human body state information according to the vital information; and after a preset interval period, (a) transmit a sensing radio wave to the body part of the user again and receive a reflection radio wave obtained by reflecting the sensing radio wave by the body part of the user. (b) process the reflection radio wave to acquire vital information of the user, and (c) determine and output human body state information according to the vital information.

As an optional solution for the smart furniture control system, the control hub is configured to receive the human body state information output by the millimeter wave detection apparatus and output a first drive signal according to a preset scenario; and the drive assembly is configured to drive smart furniture to complete a preset action according to the first drive signal.

As an optional solution for the smart furniture control system, a manual hub is further included and is connected to the control hub; the manual hub is configured to send a control signal to the control hub, the control hub is configured to send a second drive signal to the drive assembly after receiving the control signal, and the second drive signal has a higher priority than the first drive signal.

A smart sofa chair is provided. The smart sofa chair includes a sofa sitting portion, a sofa backrest, a sofa legrest, a manual controller and the smart furniture control system of any one of the preceding solutions, and the millimeter wave detection apparatus of the smart furniture control system is disposed on the sofa backrest.

A control method of a smart sofa chair is provided and performed by the preceding smart sofa chair. The control method includes using the millimeter wave detection apparatus to detect vital information of a user of the smart sofa chair and acquiring human body state information of the user according to the vital information; and analyzing the human body state information and controlling the smart sofa chair to perform a preset action according to an analysis result.

As an optional solution for the control method of the smart sofa chair, the vital information includes heart rate information, breathing information, pulse information, and a physical state of the user; and the human body state information includes a sleep state, a sedentary state, a leisure state, a tiring state, and a blank state.

As an optional solution for the control method of the smart sofa chair, in response to the user being in the sleep state, the preset action is to heat a seat cushion of the smart sofa chair by the smart sofa chair; or in response to the user being in the sleep state, the preset action is to control the; or in response to the user being in the sleep state, the preset action is to control the smart sofa chair to switch to a television (TV) posture state or a lying posture state; in response to the user being in the sedentary state, the preset action is to control the smart sofa chair to send a prompt message; and in response to the user being in a long-term lying state, the preset action is to fold at least one of the sofa backrest or the sofa legrest.

As an optional solution for the control method of the smart sofa chair, the smart sofa chair is provided with a vibration member; and the prompt message is vibration generated by the vibration member.

As an optional solution for the control method of the smart sofa chair, in response to the user performing an unexpected action in the middle of the smart sofa chair performing the preset action, the smart sofa chair is controlled to stop the preset action.

As an optional solution for the control method of the smart sofa chair, the user may send a control signal for controlling the smart sofa chair through the manual controller, and the control signal has the highest priority.

As an optional solution for the control method of the smart sofa chair, the control method of the smart sofa chair further includes sending at least one of the vital information or the human body state information to a control terminal.

As an optional solution for the control method of the smart sofa chair, the control terminal is any one of a mobile phone, a personal digital assistant (PDA), a television, a computer, or a tablet computer.

### BRIEF DESCRIPTION OF DRAWINGS

FIG. 1 is diagram one illustrating the structure of a millimeter wave detection apparatus according to an embodiment of the present application.
FIG. 2 is diagram two illustrating the structure of a millimeter wave detection apparatus according to an embodiment of the present application.
FIG. 3 is diagram three illustrating the structure of a millimeter wave detection apparatus according to an embodiment of the present application.
FIG. 4 is diagram four illustrating the structure of a millimeter wave detection apparatus according to an embodiment of the present application.
FIG. 5 is diagram five illustrating the structure of a millimeter wave detection apparatus according to an embodiment of the present application.
FIG. 6 is diagram six illustrating the structure of a millimeter wave detection apparatus according to an embodiment of the present application.
FIG. 7 is diagram seven illustrating the structure of a millimeter wave detection apparatus according to an embodiment of the present application.
FIG. 8 is diagram eight illustrating the structure of a millimeter wave detection apparatus according to an embodiment of the present application.
FIG. 9 is an exploded view of a millimeter wave detection apparatus according to an embodiment of the present application.
FIG. 10 is diagram one illustrating the structure of a millimeter wave detection apparatus mounted horizontally according to an embodiment of the present application.
FIG. 11 is diagram two illustrating the structure of a millimeter wave detection apparatus mounted horizontally according to an embodiment of the present application.
FIG. 12 is diagram one illustrating the structure of a millimeter wave detection apparatus mounted vertically according to an embodiment of the present application.
FIG. 13 is diagram two illustrating the structure of a millimeter wave detection apparatus mounted vertically according to an embodiment of the present application.
FIG. 14 is diagram one illustrating the structure of a millimeter wave detection apparatus assembled to a smart sofa chair according to an embodiment of the present application.
FIG. 15 is diagram two illustrating the structure of a millimeter wave detection apparatus assembled to a smart sofa chair according to an embodiment of the present application.
FIG. 16 is a diagram illustrating the structure of a wire according to an embodiment of the present application.
FIG. 17 is a diagram illustrating the structure of a mounting housing according to an embodiment of the present application.
FIG. 18 is an enlarged view at A in FIG. 1.
FIG. 19 is a distance test diagram of a millimeter wave sensor according to an embodiment of the present application.
FIG. 20 is a diagram of a phase angle algorithm for human breathing data measured by a millimeter wave sensor according to an embodiment of the present application.
FIG. 21 is a diagram illustrating the structure of a smart sofa chair in a sitting posture state according to an embodiment of the present application.
FIG. 22 is a diagram illustrating the structure of a smart sofa chair in a TV posture state according to an embodiment of the present application.
FIG. 23 is a diagram illustrating the structure of a smart sofa chair in a lying posture state according to an embodiment of the present application.
FIG. 24 is a diagram illustrating the structure of a smart furniture control system according to an embodiment of the present application.
FIG. 25 is a diagram illustrating an electrical control integration form in which a smart sofa chair uses a direct current (DC) 5V power supply adapter for communication according to an embodiment of the present application.
FIG. 26 is a diagram illustrating an electrical control integration form in which a smart sofa chair uses an alternating current/direct current (AC/DC) power supply for communication according to an embodiment of the present application.
FIG. 27 is a diagram illustrating an electrical control integration form in which a smart sofa chair uses a direct current/direct current (DC/DC) power supply for communication according to an embodiment of the present application.
FIG. 28 is a diagram illustrating an electrical control integration form in which a smart sofa chair uses a controller built-in power supply for communication according to an embodiment of the present application.

### Reference list

- 1: mounting portion
- 11: mounting housing
- 111: mounting surface
- 112: quick-mounting structure
- 113: wire fastener
- 114: mounting clip
- 115: mounting groove
- 116: angle scale
- 117: first through hole
- 12: circuit board
- 2: sensing portion
- 21: sensing housing
- 211: angle pointer
- 212: second through hole
- 22: millimeter wave sensor
- 23: sensing panel
- 3: mounting structure
- 31: adapter plate
- 32: mounting plate
- 4: quick-mounting member
- 5: wire
- 51: connection structure
- 6: adjustment connector
- 71: first sensor
- 72: second sensor
- 100: smart sofa chair
- 101: sofa sitting portion
- 102: sofa backrest
- 103: sofa legrest
- 104: backrest drive unit
- 105: legrest drive unit
- 106: manual controller

### DETAILED DESCRIPTION

Technical solutions in embodiments of the present application are described in conjunction with drawings in the embodiments of the present application. Apparently, the embodiments described herein are part of the embodiments of the present application.

Therefore, the following description of the embodiments of the present application shown in the drawings is not intended to limit the claimed scope of the present application and only illustrates selected embodiments of the present application. Based on the embodiments of the present application, all other embodiments obtained by those of ordinary skill in the art without creative work are within the scope of the present application.

It is to be noted that similar reference numerals and letters indicate similar items in subsequent drawings. Therefore, once a certain item is defined in one drawing, the item needs no more further definition and explanation in the subsequent drawings.

In the description of the present application, orientations or position relations indicated by terms such as "upper", "lower", "left", "right", "vertical", "horizontal", "in", and "out" are based on the drawings or those of the product of the present application usually placed during use. These orientations or position relations are intended only to facilitate and simplify the description of the present application and not to indicate or imply that an apparatus or element referred to must have such particular orientations or must be configured or operated in such particular orientations. Thus, these orientations or position relations are not to be construed as limiting the present application. Moreover, terms such as "first", "second", and "third" are used only for distinguishing description and are not to be construed as indicating or implying relative importance. In the description of the present application, unless otherwise noted, the term "a plurality of" or "multiple" means two or more.

In the description of the present application, it is also to be noted that terms "disposed" and "connected" are to be understood in a broad sense unless otherwise expressly specified and limited. For example, the term "connected" may refer to "securely connected", "detachably connected", or "integrated" or may refer to "mechanically connected" or "electrically connected". For those of ordinary skill in the art, meanings of the preceding terms in the present application may be understood based on situations.

In the present application, unless otherwise expressly specified and limited, when a first feature is described as being "on" or "below" a second feature, the first feature and the second feature may be in direct contact or be in contact via another feature between the two features instead of being in direct contact. Moreover, when the first feature is described as being "on", "above", or "over" the second feature, the first feature is right on, above, or over the second feature, the first feature is obliquely on, above, or over the second feature, or the first feature is simply at a higher level than the second feature. When the first feature is described as being "under", "below", or "underneath" the second feature, the first feature is right under, below, or underneath the second feature, the first feature is obliquely under, below, or underneath the second feature, or the first feature is simply at a lower level than the second feature.

The embodiments of the present application are described below. Examples of the embodiments are shown in the drawings, where the same or similar reference numerals indicate the same or similar elements or elements having the same or similar functions. The embodiments described below with reference to the drawings are illustrative, used only for explaining the present application, and not to be construed as limiting the present application.

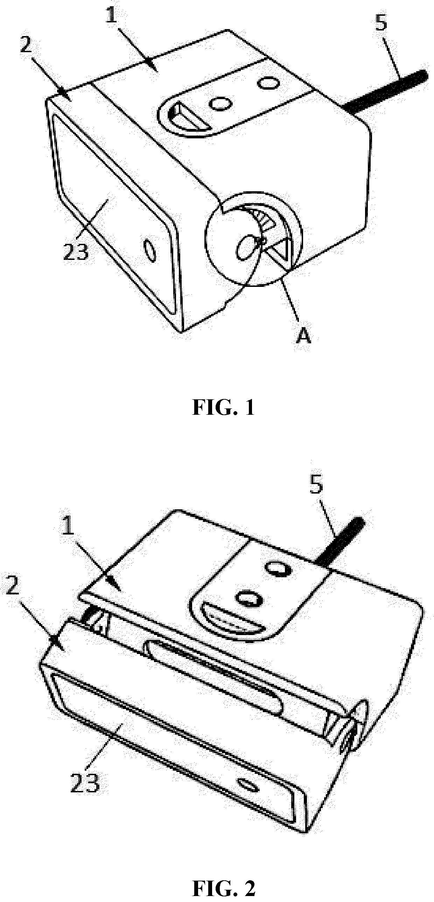

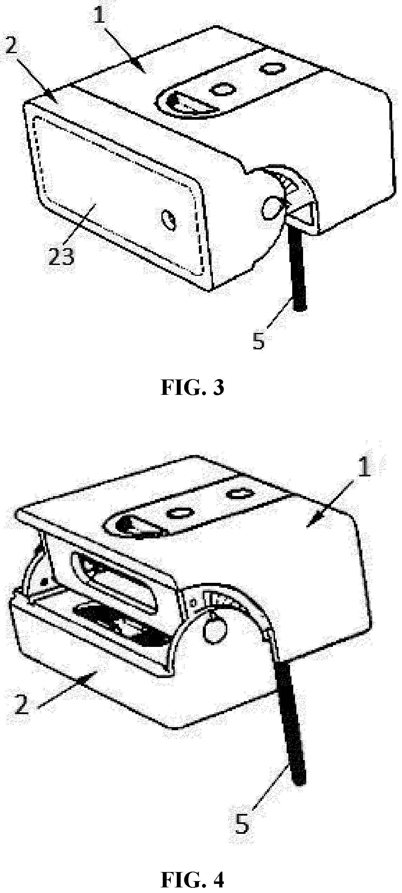

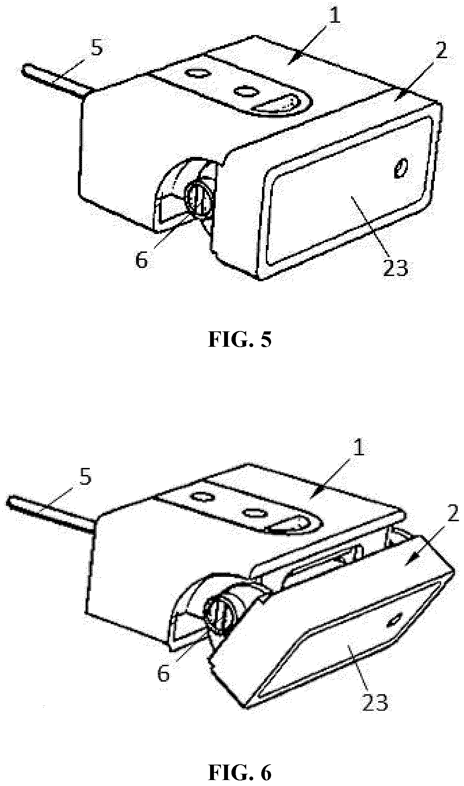

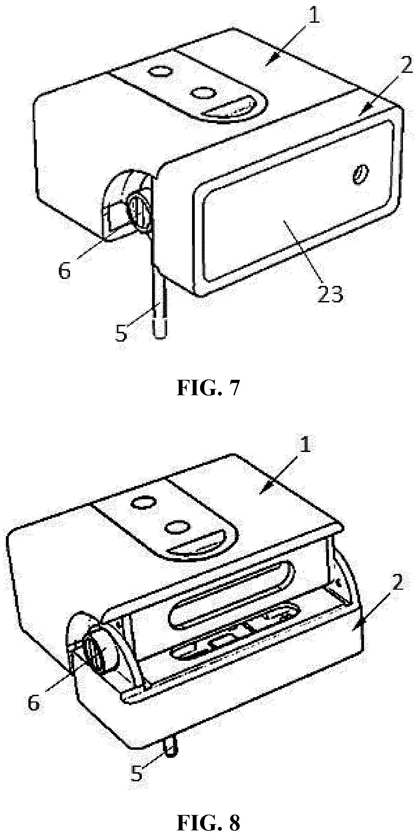

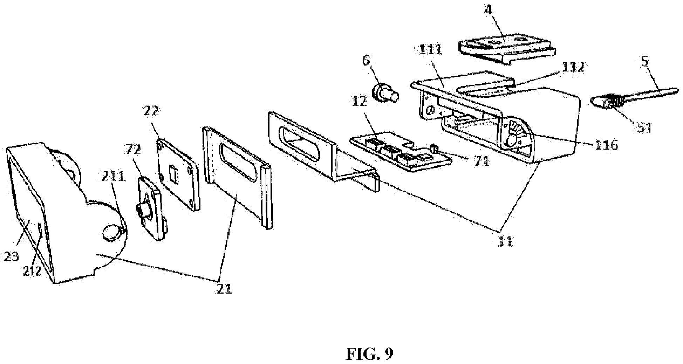

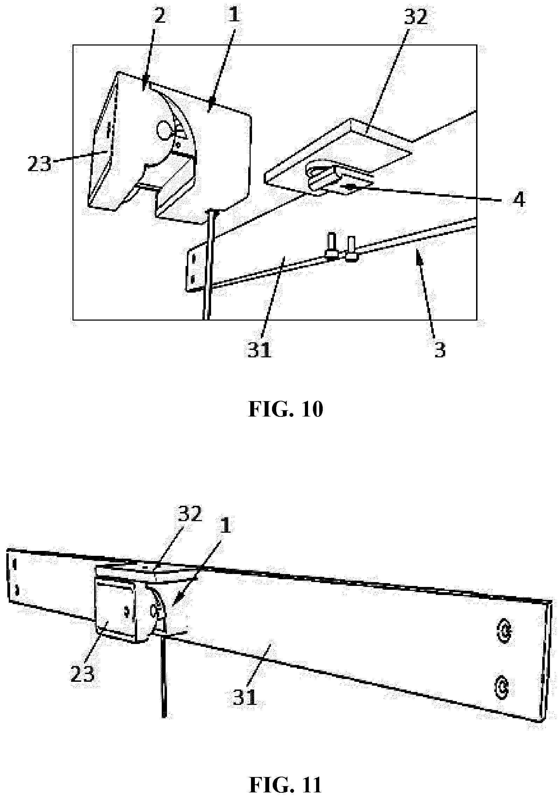

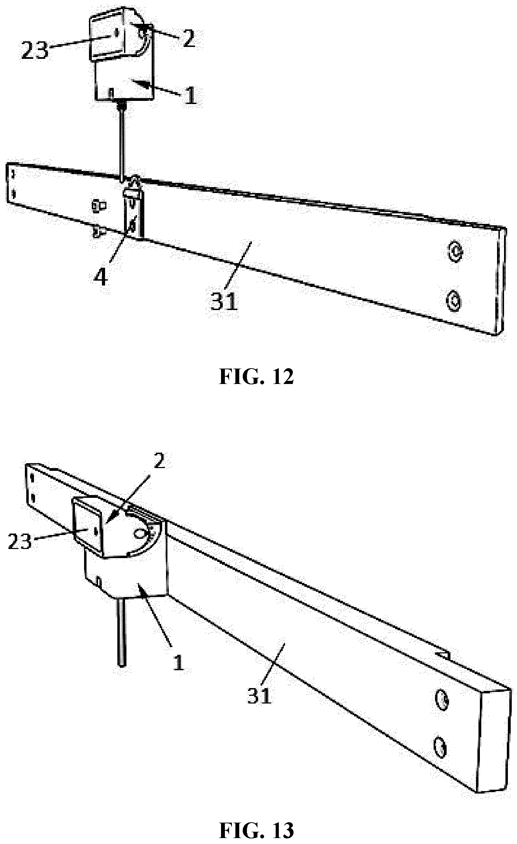

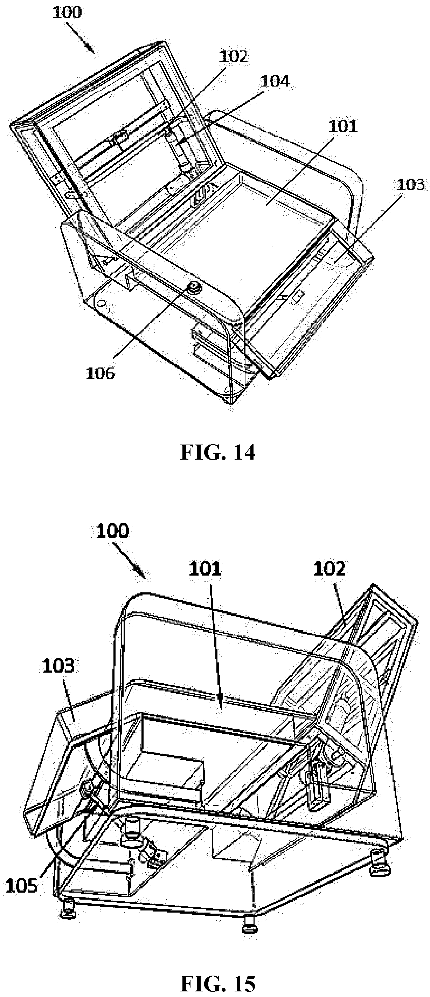

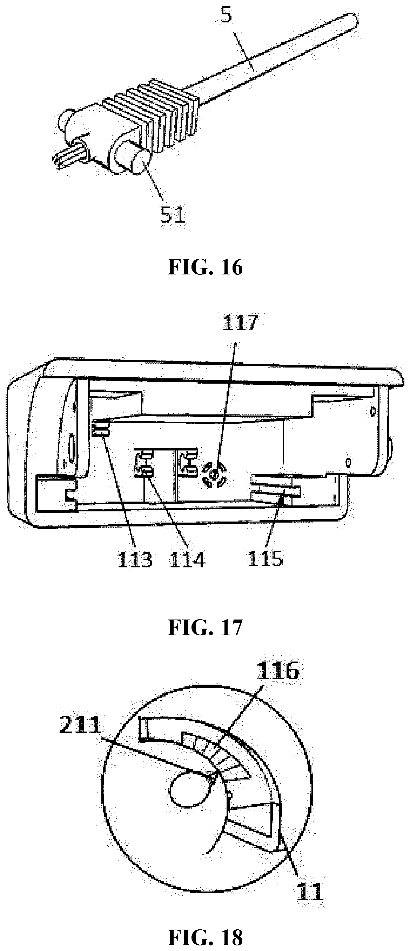

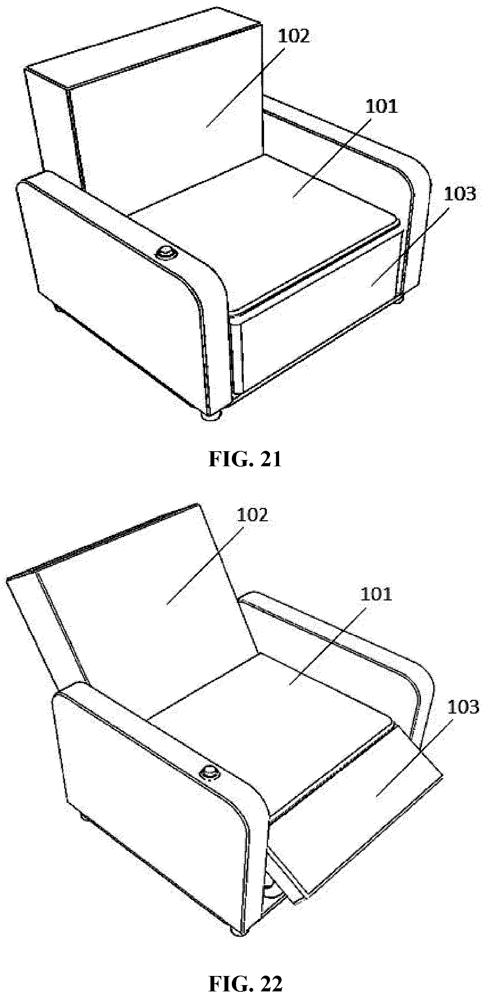

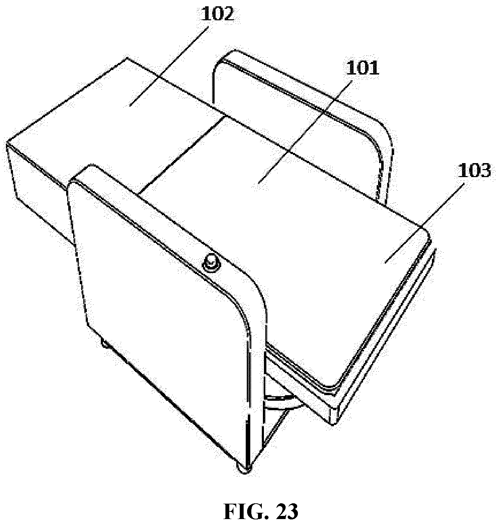

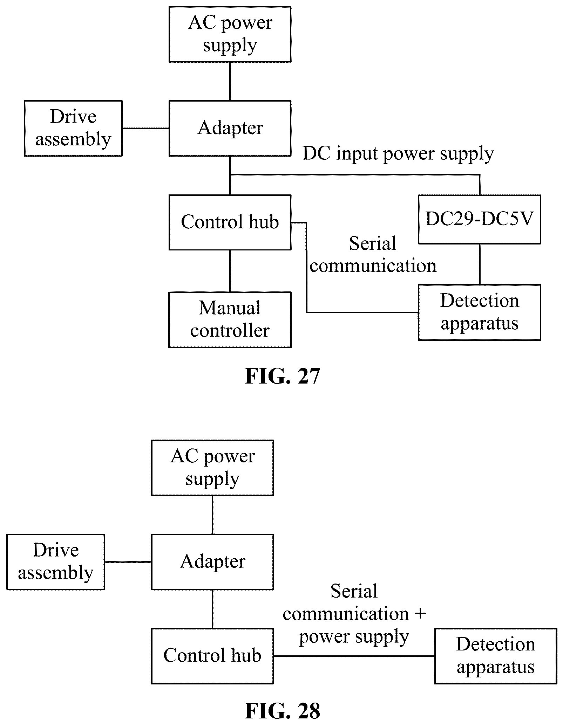

As shown in FIGS. 1 to 28, this embodiment provides a smart sofa chair 100. The smart sofa chair 100 includes a sofa sitting portion 101, a sofa backrest 102, a sofa legrest 103, a manual controller 106, and a millimeter wave detection apparatus disposed on the sofa backrest 102. Vital information of a user may be acquired through the millimeter wave detection apparatus to improve the intelligentization degree of the smart sofa chair 100. Optionally, the millimeter wave detection apparatus is arranged corresponding to the chest of the user to improve the detection accuracy.

In this embodiment, the millimeter wave detection apparatus includes a mounting portion 1 and a sensing portion 2. The mounting portion 1 includes a mounting housing 11 and a circuit board 12 disposed within the mounting housing 11. The sensing portion 2 includes a sensing housing 21 and a millimeter wave sensor 22 disposed within the sensing housing 21. The millimeter wave sensor 22 is electrically connected to the circuit board 12. The sensing housing 21 is provided with a sensing panel 23 corresponding to the millimeter wave sensor 22. The sensing housing 21 is disposed on the mounting housing 11 with an adjustable angle to adjust the orientation of the sensing panel 23.

When the millimeter wave detection apparatus is mounted, the mounting housing 11 is securely installed on the backrest of the sofa chair, and the sensing housing 21 is disposed on the mounting housing 11 with an adjustable angle so that the orientation of the sensing panel 23 can be adjusted according to actual requirements, thereby improving the matching degree between the millimeter wave detection apparatus and the smart sofa chair 100 and facilitating an improvement in the detection accuracy of the millimeter wave sensor 22. In this manner, the millimeter wave sensor 22 can determine whether the user is located on the smart sofa chair 100. If the user is located on the smart sofa chair 100, the user's heart rate, breathing, pulse, sleep state (including a low, medium, or deep sleep state), fatigue, and other vital information can be accurately acquired, and the impact of electromagnetic radiation on human health can be reduced.

Optionally, the sensing panel 23 is mounted facing the chest of the user to enable millimeter wave radar to face the chest of the user so that the accuracy of detection data can be effectively improved.

Optionally, the angle adjustment range of the sensing housing 21 is from 0° to 90°, and the adjustment range is large and is applicable to the smart sofa chair 100 in any form.

In this embodiment, the mounting housing 11 is provided with a mounting surface 111. In the first extreme state, the included angle between the sensing panel 23 and the mounting surface 111 is 90°. In the second extreme state, the included angle between the sensing panel 23 and the mounting surface 111 is 180°. In other words, when the sensing panel 23 flips from the first extreme state to the second extreme state, the flipping angle is 90°.

Optionally, the millimeter wave detection apparatus further includes a wire 5 electrically connected to the circuit board 12. To match the wire 5 with the adjustable sensing portion 2 and avoid excessively pulling the wire 5, the exterior of the wire 5 is securely provided with a connection structure 51. The connection structure 51 is rotatably connected to the mounting housing 11. For one aspect, the positioning function can be provided for the wire 5; and for another aspect, the wire 5 can be enabled to swing at an appropriate angle as the sensing portion 2 flips, thereby reducing the force applied to the wire 5.

Optionally, a wire fastener 113 is disposed within the mounting housing 11 and can prevent the wire 5 from falling off. The wire fastener 113 secures the wire 5, which can improve the connection stability between the wire 5 and the circuit board 12. Exemplarily, the wire fastener 113 is a wire clip, and the wire clip is used for clamping the wire 5 to secure the wire 5 and easily dismantle or mount the wire 5.

In this embodiment, the connection structure 51 on the exterior of the wire 5 is a cylinder, and a mounting clip 114 is disposed within the mounting housing 11. The mounting clip 114 is provided with an opening through which the connection structure 51 may snap into the mounting clip 114, which is convenient for assembly and can also prevent the connection structure 51 from falling out of the opening, and the cylindrical connection structure 51 can rotate freely relative to the mounting clip 114.

A mounting groove 115 is further disposed within the mounting housing 11, and the circuit board 12 is inserted into the mounting groove 115. The mounting groove 115 plays the positioning function for the circuit board 12 to prevent the circuit board 12 from displacement, and the mounting is convenient.

Optionally, a first side of the mounting housing 11 is provided with an angle scale 116, and an angle pointer 211 corresponding to the angle scale 116 is provided at the same side of the sensing housing 21 as the angle scale 116. After the sensing housing 21 is connected to the mounting housing 11, the angle pointer 211 points to an angle value; when the sensing housing 21 is flipped, the flipping angle of the sensing housing 21 may be read through the angle pointer 211 and the angle scale 116.

Optionally, a second side of the mounting housing 11 relative to the angle scale 116 is provided with a detachable adjustment connector 6. The sensing housing 21 can rotate relative to the mounting housing 11, and the adjustment connector 6 is configured to lock or unlock the sensing housing 21 and the mounting housing 11. When the orientation of the sensing panel 23 requires adjustment, it is only necessary to unlock the adjustment connector 6, flip the sensing housing 21, adjust the sensing panel 23 to the target orientation, and then use the adjustment connector 6 to lock the sensing housing 21 and the mounting housing 11, which is convenient to operate.

In this embodiment, the adjustment connector 6 is a bolt. The head of a first end of the bolt abuts against the sensing housing 21, and a second end of the bolt passes through the sensing housing 21 and is threadedly connected to the mounting housing 11. The bolt is tightened to lock the sensing housing 21 and the mounting housing 11; the bolt is loosened to adjust the angle of the sensing housing 21. The structure is simple and easy to operate.

Optionally, the millimeter wave detection apparatus further includes multiple function extension sensors that are mounted within the sensing housing 21 and/or the mounting housing 11. Functions of the millimeter wave detection apparatus may be added by the multiple function extension sensors. Exemplarily, the multiple function extension sensors may be temperature sensors and others.

Optionally, the multiple function extension sensors include a first sensor 71 such as a temperature sensor; the first sensor 71 is mounted within the mounting housing 11, and the mounting housing 11 is provided with a first through hole 117 corresponding to the first sensor 71 so that the temperature sensor can accurately sense the environmental temperature through the first through hole 117.

Optionally, the multiple function extension sensors include a second sensor 72 such as a pressure sensor; the second sensor 72 is mounted within the sensing housing 21, and the sensing housing 21 is provided with a second through hole 212 corresponding to the second sensor 72 so that the pressure sensor can sense the pressure of the user leaning against the backrest of the sofa chair through the second through hole 212.

Optionally, the mounting housing 11 and the sensing housing 21 each include two detachably connected housing bodies. The two housing bodies are connected to form the closed mounting housing 11 or the closed sensing housing 21 to protect the internal structure of the mounting housing 11 or the sensing housing 21. The internal structure can be dismantled and mounted or repaired by dismounting the two housing bodies.

Optionally, to facilitate the quick mounting of the millimeter wave detection apparatus, the mounting housing 11 is provided with a quick-mounting structure 112 that is configured to secure the millimeter wave detection apparatus. Exemplarily, the quick-mounting structure 112 is a slot disposed on the mounting housing 11. Certainly, the quick-mounting structure 112 may also be an insertion block disposed on the mounting housing 11 to quickly mount the millimeter wave detection apparatus in an insertion manner

The millimeter wave detection apparatus is embedded into the sofa backrest 102 of the smart sofa chair 100, and the mounting space is limited. In this embodiment, to mount the millimeter wave detection apparatus horizontally, reduce the space occupied by the millimeter wave detection apparatus in the vertical direction, avoid interference, and better secure the millimeter wave detection apparatus to the sofa backrest 102 of the smart sofa chair 100, a mounting structure 3 is also designed. The mounting structure 3 includes an adapter plate 31 and a mounting plate 32. The mounting plate 32 is securely disposed at one end of the adapter plate 31 in the width direction, and the mounting housing 11 is detachably disposed on the mounting plate 32. The mounting plate 32 is roughly parallel to the horizontal plane, and the mounting surface 111 is parallel to the mounting plate 32.

In other embodiments, the millimeter wave detection apparatus may also be mounted vertically to reduce the space occupied by the millimeter wave detection apparatus in the horizontal direction and avoid interference, so the mounting structure 3 only includes the adapter plate 31, and the mounting housing 11 is detachably disposed on the adapter plate 31. The adapter plate 31 is roughly vertical to the horizontal plane, and the mounting surface 111 is parallel to the adapter plate 31.

Optionally, the mounting structure 3 further includes a quick-mounting member 4. The quick-mounting member 4 is securely connected to the mounting structure 3 and configured to mate with the quick-mounting structure 112 on the mounting housing 11 to secure the millimeter wave detection apparatus to the mounting structure 3. In this embodiment, the quick-mounting structure 112 can be inserted into the quick-mounting member 4, and this insertion manner has a simple structure and achieves quick dismantling and mounting.

In the millimeter wave detection apparatus, the furniture control system, and the sofa chair that are provided in the present application, when the millimeter wave detection apparatus is mounted, the mounting housing is secured, the sensing housing is disposed on the mounting housing with an adjustable angle, and the orientation of the sensing panel can be adjusted according to actual requirements, which has a higher matching degree, is also conducive to an improvement in the detection accuracy of the millimeter wave sensor, and can reduce the impact of the electromagnetic radiation on the human health.

The detection principle of the millimeter wave sensor 22 is described below.

In this embodiment, the antenna transmission angle of the millimeter wave sensor 22 is 40° in the vertical direction and 80° in the horizontal direction, which is similar to the shape of an elliptical cone.

The millimeter wave sensor 22 has an approximate detection range of 0.15 m to 3 m and detects the chest and back of a human body, including the contraction and heaving of the lungs.

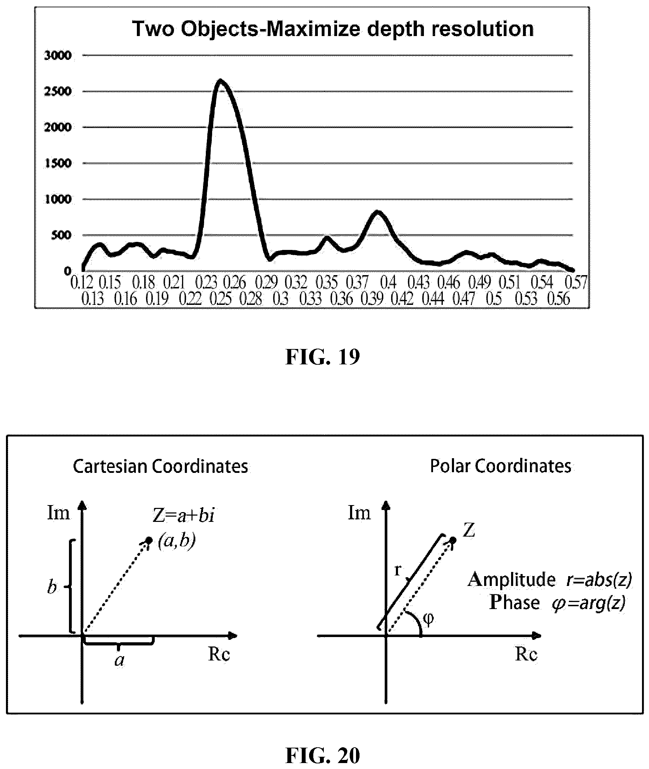

Exemplarily, FIG. 19 is a distance test diagram of the millimeter wave sensor 22, the horizontal coordinate denotes the distance and the vertical coordinate denotes the signal reflection intensity. It can be seen from FIG. 19 that there are two wave peaks. This is because two objects are detected at different distances. The distance of the first object is 0.25 m, and the distance of the second object is 0.39 m.

It is to be noted that the millimeter wave sensor 22 includes a detection assembly and a system wafer, a transceiver system of the detection assembly is responsible for transmitting a radio wave, receiving a reflection wave, and processing raw data into breathing, heart rate, and others; the system wafer is responsible for converting the breathing, the heart rate, and the others into a specific state such as the sleep state (that is, the user is in the sleep state) through a deep learning algorithm. FIG. 20 is a diagram of a phase angle algorithm for human breathing data measured by the millimeter wave sensor 22. This is the original phase data output by the millimeter wave sensor 22, which is mainly used for micro-motion detection of the breathing and heart rate. A phase signal with a coordinate phase difference of 90° is shown in FIG. 20. The signal consists of a complex number: Z = a + bi, with a and b as two real numbers. In the right figure, r denotes the intensity of the signal, and the included angle φ from the X-axis is the phase angle.

This embodiment further provides a smart furniture control system based on a millimeter wave detection apparatus. The smart furniture control system includes a control hub, a drive assembly, and the preceding millimeter wave detection apparatus, and the millimeter wave detection apparatus is configured to perform the following steps.
a. A sensing radio wave is transmitted to a body part of the user, and a reflection radio wave obtained by reflecting the sensing radio wave by the body part of the user is received.
b. The reflection radio wave is processed to acquire vital information of the user.
c. Human body state information is determined and output according to the vital information of the user.

It is to be noted that the vital information includes heart rate, breathing rate, pulse, body temperature, and others. The human body state information of the user includes the sleep state (including the low, medium, or deep sleep state), a sedentary state (that is, the user maintains a sitting posture for a preset period), a long-term lying state (that is, the user maintains a TV posture or a lying posture for a preset period and is not in the sleep state), a tiring state, and a blank state (that is, when the state of the user cannot be determined or does not belong to any of the preceding states).
d. After a preset interval period is reached, steps a to c are repeated.

In actual operation, the millimeter wave detection apparatus repeats the measurement at regular intervals to confirm that the user is still in the same human body state (such as the sedentary state) or to promptly detect a change in the state of the user (such as the user moves).

Optionally, the control hub is configured to receive the human body state information output by the millimeter wave detection apparatus and output a first drive signal according to a preset scenario. Optionally, the drive assembly is configured to drive smart furniture to complete a preset action according to the first drive signal.

Optionally, the smart furniture control system further includes a manual hub connected to the control hub; the user may control the drive assembly to make an action the user wants through the manual hub. The user sends a control signal to the control hub through the manual hub, and the control hub is configured to send a second drive signal to the drive assembly according to the control signal. The second drive signal has a higher priority than the first drive signal. The manual hub is directly controlled by the user and may interrupt signals sent by the millimeter wave detection apparatus and the control hub. For example, if the millimeter wave detection apparatus determines that the user is in the sleep state, the control hub sends the first drive signal to change the sofa to a lying posture, but the user wakes up halfway between changing the sofa to the lying posture, the drive signal of changing the sofa to the lying posture may be interrupted through the manual hub, and the second drive signal sent by the user through the manual hub is executed.

To improve the intelligentization degree of the smart sofa chair 100, this embodiment further provides a control method of the smart sofa chair that is performed by the preceding smart sofa chair 100. The control method of the smart sofa chair includes the following: in S1, using the millimeter wave detection apparatus to detect vital information of a user of the smart sofa chair 100 and acquiring human body state information of the user according to the vital information of the user; and in S2, analyzing the human body state information and controlling the smart sofa chair 100 to perform a preset action according to an analysis result.

The vital information of the user is acquired through the millimeter wave detection apparatus, and the human body state information of the user is acquired by analyzing and calculating the acquired vital information so that the smart sofa chair 100 can be automatically controlled according to the preset action, thereby improving the intelligentization degree of the smart sofa chair 100 and the comfort of the user.

The vital information of the user is collected so that the requirements of the user can be determined by analyzing the vital information to automatically control the smart sofa chair 100 and improve the comfort of the user.

Optionally, the user may preset the preset action of the smart sofa chair 100 before using the smart sofa chair 100 or may also select the factory settings.

Optionally, if the user is in the sleep state, the preset action is that the smart sofa chair 100 heats a seat cushion of the smart sofa chair 100 to prevent the user from catching a cold.

Certainly, if the user is in the sleep state, the preset action may also be configured to control the smart sofa chair 100 to switch to a TV posture state or a lying posture state to improve the rest comfort of the user.

If the user is in the low sleep state or the medium sleep state, the smart sofa chair 100 is controlled to switch to the TV posture state. In the TV posture state, the sofa backrest 102 of the smart sofa chair 100 has two states of being tilted by 30° and being tilted by 60°. When the user is in the low sleep state, the sofa backrest 102 of the smart sofa chair 100 may also be controlled to be tilted by 30°; and when the user is in the medium sleep state, the sofa backrest 102 of the smart sofa chair 100 is controlled to be tilted by 60°. If the user is in the deep sleep state, the smart sofa chair 100 is controlled to switch to the lying posture state.

Optionally, if the user is in the sedentary state (that is, the user maintains the sitting posture for more than the preset period), the preset action is to control the smart sofa chair 100 to send a prompt message to remind the user to stand up and stretch the muscles to maintain a healthy human blood circulation function; the preset action can also prevent the user from watching TV for too long, avoid visual fatigue, and prevent myopia.

Optionally, if the user is in the long-term lying state, the preset action is to fold the sofa backrest 102 and/or the sofa legrest 103 to remind the user to change the posture and maintain the healthy human blood circulation function; the preset action can also prevent the user from watching TV for too long, avoid visual fatigue, and prevent myopia.

In an embodiment, the smart sofa chair 100 is provided with a vibration member; and the prompt message is the vibration generated by the vibration member.

It is to be noted that the user may also set reminders such as cooking and boiling water so that after using the smart sofa chair 100 for a set period, the user can be promptly reminded to start processing the reminders to avoid forgetting.

The following steps are further included when the smart sofa chair 100 is controlled to perform the preset action.

If the user makes an unexpected action, the smart sofa chair 100 is controlled to stop the preset action. The unexpected action refers to any action that may make the millimeter wave detection apparatus determine that the user has left the current human body state, including, but not limited to, raising a hand, falling, and a sudden increase in heart rate.

Exemplarily, if the user is in the sleep state, the smart sofa chair 100 is controlled to switch to the TV posture state or the lying posture state. During the switching process, if the user raises a hand or makes other actions, it indicates that the sleep state is ended, the user suddenly wakes up, or the sleep state is wrongly determined. In this case, the smart sofa chair 100 is controlled to stop switching to the TV posture state or the lying posture state.

Certainly, the unexpected action may also refer to any operation of the manual controller 106 of the smart sofa chair 100.

The user may send a control signal for controlling the smart sofa chair 100 through the manual controller 106, and this control signal has the highest priority.

Optionally, the control method of the smart sofa chair further includes sending the vital information and/or the human body state information to a control terminal.

This arrangement may allow the user or the family member of the user to check vital information records through the control terminal and know their own health conditions.

Optionally, the control terminal is any one of a mobile phone, a personal digital assistant (PDA), a television, a computer, or a tablet computer. Further, a control program (such as an application program (APP)) of the smart sofa chair 100 is pre-installed in the control terminal, and the user may control the smart sofa chair 100 through the pre-installed control program.

A warning reminder may also be configured on the control terminal to promptly remind the user to pay attention to the health condition when the vital information of the user exceeds the preset value.

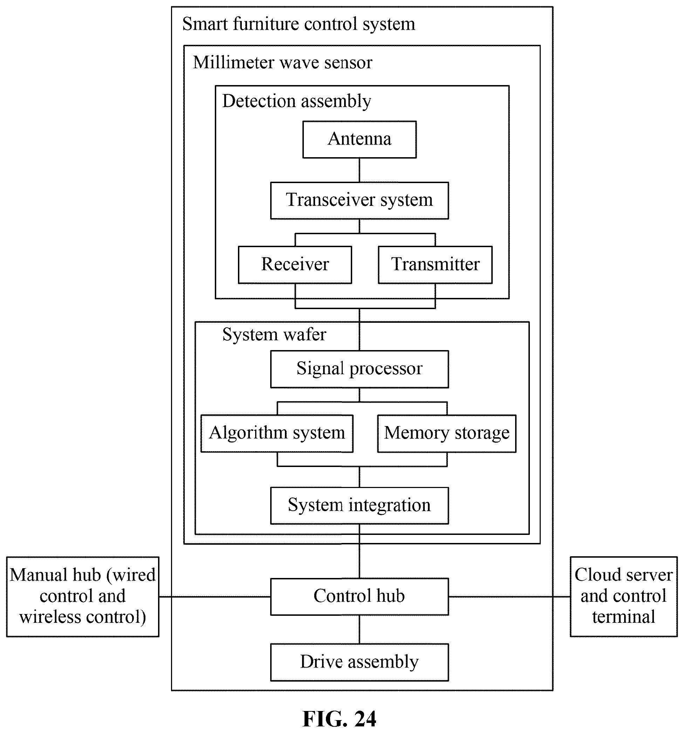

Exemplarily, the diagram illustrating the structure of the smart furniture control system based on the millimeter wave detection apparatus is shown in FIG. 24.

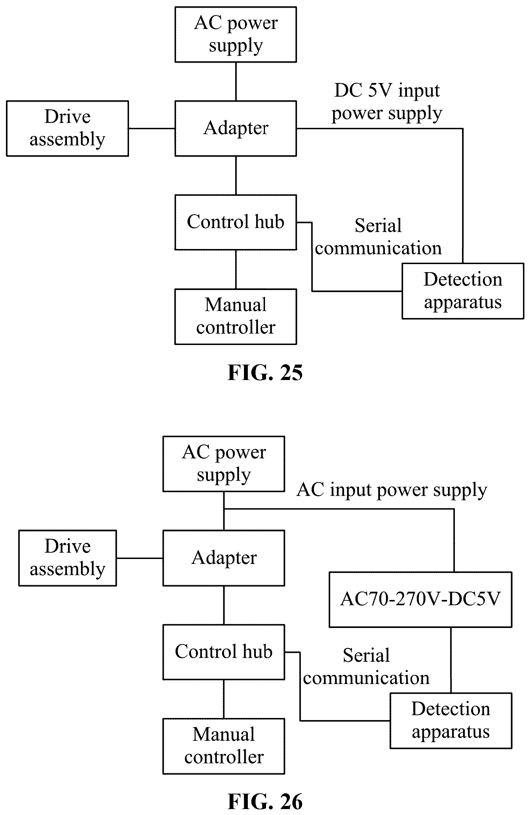

Exemplarily, the electrical control integration form of the smart sofa chair 100 may be any form shown in FIGS. 25 to 28 and configured and applied according to solutions and requirements.

The control method of the smart sofa chair provided in the present application is applied to the preceding smart sofa chair, which improves the intelligentization degree of the smart sofa chair and satisfies the requirements of the user.

It is to be noted that the "priority" in the preceding embodiments refers to the execution selection of an execution unit (such as the drive assembly) when the execution unit receives different instructions (or commands, or control signals, or drive signals). If the execution unit (such as the drive assembly, similarly hereinafter) receives different instructions (or the commands, or the control signals, or the drive signals, similarly hereinafter) at the same time, the execution unit only executes the instruction with a higher priority; if the execution unit receives another instruction while executing the received instruction, the execution unit only executes the instruction with a high priority, or stops the instruction with a low priority and returns to the state before execution, and then executes the instruction with the high priority.

## Claims

1. A millimeter wave detection apparatus, comprising:
a mounting portion (1) comprising a mounting housing (11) and a circuit board (12) disposed within the mounting housing (11); and
a sensing portion (2) comprising a sensing housing (21) and a millimeter wave sensor (22) disposed within the sensing housing (21), wherein the millimeter wave sensor (22) is electrically connected to the circuit board (12), the sensing housing (21) is provided with a sensing panel (23) corresponding to the millimeter wave sensor (22), and the sensing housing (21) is disposed on the mounting housing (11) with an adjustable angle to adjust an orientation of the sensing panel (23).

2. The millimeter wave detection apparatus of claim 1, wherein an angle adjustment range of the sensing housing (21) is from 0° to 90°.

3. The millimeter wave detection apparatus of claim 2, wherein a first side of the mounting housing (11) is provided with an angle scale (116), and an angle pointer (211) corresponding to the angle scale (116) is provided on a same side of the sensing housing (21) relative to the angle scale (116); and
a second side of the mounting housing (11) relative to the angle scale (116) is provided with a detachable adjustment connector (6), wherein the adjustment connector (6) is configured to lock or unlock the sensing housing (21) and the mounting housing (11).

4. The millimeter wave detection apparatus of claim 1, further comprising a wire (5) electrically connected to the circuit board (12), wherein a connection structure (51) is fixed at an exterior of the wire (5), and the connection structure (51) is rotatably connected to the mounting housing (11); and
a wire fastener (113) is disposed within the mounting housing (11) and configured to prevent the wire (5) from falling off.

5. The millimeter wave detection apparatus of claim 1, further comprising a plurality of function extension sensors, wherein the plurality of function extension sensors are installed within at least one of the sensing housing (21) or the mounting housing (11).

6. The millimeter wave detection apparatus of claim 5, wherein the plurality of function extension sensors comprise at least one of:
a first sensor (71) installed within the mounting housing (11), wherein the mounting housing (11) is provided with a first through hole (117) corresponding to the first sensor (71); or
a second sensor (72) installed within the sensing housing (21), wherein the sensing housing (21) is provided with a second through hole (212) corresponding to the second sensor (72).

7. The millimeter wave detection apparatus of claim 1, wherein the mounting housing (11) is provided with a quick-mounting structure (112), wherein the quick-mounting structure (112) is configured to secure the millimeter wave detection apparatus.

8. A mounting structure, configured to secure the millimeter wave detection apparatus of any one of claims 1 to 7, wherein the mounting structure (3) comprises:
an adapter plate (31) and a mounting plate (32), wherein the mounting plate (32) is securely disposed at one end of the adapter plate (31) in a width direction, and the mounting housing (11) is detachably disposed on the mounting plate (32); or
wherein the mounting structure (3) comprises an adapter plate (31), and the mounting housing (11) is detachably disposed on the adapter plate (31).

9. The mounting structure of claim 8, wherein the mounting structure (3) is provided with a quick-mounting member (4), wherein the quick-mounting member (4) is securely connected to the mounting structure (3) and configured to mate with a quick-mounting structure (112) on the mounting housing (11) to secure the millimeter wave detection apparatus to the mounting structure (3).

10. A smart furniture control system, comprising a control hub, a drive assembly, and the millimeter wave detection apparatus of any one of claims 1 to 7, wherein the millimeter wave detection apparatus is configured to perform the following operations:
transmitting a sensing radio wave to a body part of a user and receiving a reflection radio wave obtained by reflecting the sensing radio wave by the body part of the user;
processing the reflection radio wave to acquire vital information of the user;
determining and outputting human body state information according to the vital information; and
after a preset interval period, transmitting a sensing radio wave to the body part of the user again and receiving a reflection radio wave obtained by reflecting the sensing radio wave by the body part of the user; processing the reflection radio wave to acquire vital information of the user; and determining and outputting human body state information according to the vital information.

11. The smart furniture control system of claim 10, wherein the control hub is configured to receive the human body state information output by the millimeter wave detection apparatus and output a first drive signal according to a preset scenario; and the drive assembly is configured to drive smart furniture to complete a preset action according to the first drive signal.

12. The smart furniture control system of claim 11, further comprising a manual hub, wherein the manual hub is connected to the control hub and configured to send a control signal to the control hub, and the control hub is configured to send a second drive signal to the drive assembly after receiving the control signal, wherein the second drive signal has a higher priority than the first drive signal.

13. A smart sofa chair, comprising a sofa sitting portion (101), a sofa backrest (102), a sofa legrest (103), a manual controller (106), and the smart furniture control system of any one of claims 10 to 12, wherein the millimeter wave detection apparatus of the smart furniture control system is disposed on the sofa backrest (102).

14. A control method of a smart sofa chair, being performed by the smart sofa chair (100) of claim 13 and comprising:
detecting, by the millimeter wave detection apparatus, vital information of a user of the smart sofa chair (100), and acquiring human body state information of the user according to the vital information; and
analyzing the human body state information, and controlling, according to an analysis result, the smart sofa chair (100) to perform a preset action.

15. The control method of claim 14, wherein the vital information comprises heart rate information, breathing information, pulse information, and a physical state of the user; and
the human body state information comprises a sleep state, a sedentary state, a leisure state, a tiring state, and a blank state.

16. The control method of claim 15, wherein in response to the user being in the sleep state, the preset action is that the smart sofa chair (100) heats a seat cushion of the smart sofa chair (100); or in response to the user being in the sleep state, the preset action is that the smart sofa chair (100) is controlled to switch to a television, TV, posture state or a lying posture state;
in response to the user being in the sedentary state, the preset action is that the smart sofa chair (100) is controlled to send a prompt message; and
in response to the user being in a long-term lying state, the preset action is that at least one of the sofa backrest (102) or the sofa legrest (103) is folded.

17. The control method of claim 16, wherein the smart sofa chair (100) is provided with a vibration member; and
the prompt message is vibration generated by the vibration member.

18. The control method of claim 16, comprising:
in response to the user performing an unexpected action in the middle of the smart sofa chair (100) performing the preset action, controlling the smart sofa chair (100) to stop the preset action.

19. The control method of claim 16, wherein the user is capable of sending a control signal for controlling the smart sofa chair (100) through the manual controller (106), wherein the control signal has a highest priority.

20. The control method of claim 14, further comprising:
sending at least one of the vital information or the human body state information to a control terminal.

21. The control method of claim 20, wherein the control terminal is any one of a mobile phone, a personal digital assistant, PDA, a television, a computer, or a tablet computer.
